# EUROPEAN PATENT APPLICATION

(11) **EP 3 584 743 A1**
(43) Date of publication of application: **25.12.2019**
(21) Application number: 18382434.1
(22) Date of filing: 18.06.2018
(51) Int. Cl.: G06K 9/00, A61B 5/024, G06F 21/32

(54) **IMPROVED BIOMETRIC USER'S AUTHENTICATION**

(71) Applicant: Telefonica S.A., 28013 Madrid (ES)
(72) Inventor: SEGURA PERALES, Carlos, 28013 MADRID (ES); LUQUE SERRANO, Jordi, 28013 MADRID (ES); ESTEBAN ZARZA, Javier, 28013 MADRID (ES); FABREGAT SERRA, Joan, 28013 MADRID (ES); MARAVILLA GIRBES, Alexandre, 28013 MADRID (ES); BIANZINO, Aruna, Prem, 28013 MADRID (ES); DE LOS SANTOS, Sergio, 28013 MADRID (ES)
(74) Representative: Herrero & Asociados, S.L.

(57) **Abstract**

It is provided a method, device and system for providing authentication of an user of an electronic device (for example, when using a certain service or accessing a certain asset or premises). The user's authentication is based on the measurement of a biometric/biotechnological feature of the individual (preferably on the user's pulse) and on the detection of the user motion (and eventually location) status change.

## Description

### TECHNICAL FIELD OF THE INVENTION

Present invention generally relates to security in services provided to electronic device users in a communications network using biometric features. More specifically, it relates to continuous authentication of electronic device (e.g. mobile communications device) users based on user's biometric (e.g. pulse) measurement where other factors as the movement and the location of the user may also be taken into account.

### BACKGROUND OF THE INVENTION

Mobile communication devices (also called mobile devices), such as cellular or mobile telephones, tablets, i-pads, notebooks...are everyday more and more used. And these devices are used not only to place telephone calls but for an enormous range of services. The use of mobile communication devices to provide an everyday wider range of services makes essential a security environment where the mobile device user (to which the service is provided) is univocally identified, in order to ensure that the service is provided to the correct user and not to an unauthorized user.

The need of identifying users consuming different types of services or accessing different types of assets or premises is closely related to the prevention of identity theft but privacy security must also be taken into account. It is therefore of extreme interest to establish whether users are who they claim to be, but it is as much important to keep confidentiality about the exchanged information in such process.

The user identification process is usually based on some credentials, whose ownership and verification guarantee to check the user identity. The used credentials are usually emitted by a trusted authority and are theoretically impossible (or at least extremely difficult) to falsify, allowing to determine whether the users corresponds to who they claim to be or not.

The user identification process is built on top of the so called validation or authentication mechanisms. Such mechanisms are designed to validate some information that the user brings in order to access the requested services (e.g. digital services). The carried information (which the user provides to the authentication mechanism to be validated) usually fall into one of the following categories: something that the user knows (e.g., a secret keyword), something that the user owns (e.g., an physical object also called physical token, like a smartcard, a SIM card, an NFC tag...), something that the owner is (e.g., any measurable physical feature, univocally identifying the user, like fingerprints or iris identification), something that the user does (e.g., motion patterns), or somewhere that the user is (e.g., being in a specific location). This information is what is known as identifier or digital credential. Sometimes a combination of information belonging to different of these categories is used in order to improve security. Each kind of information used in the process is referred to as "authentication factor" or "identification factor".

The effectiveness of any authentication solution cannot be measured only in terms of security - as many of them are equivalent on that aspect - but rather accounting also for usability, ease of implementation, resource and energy consumption, cost.... Only by keeping into account all these aspects at the same time it is possible to obtain a wide adoption of the authentication solution by the users.

The current authentication methods are keen to some major problems, as for example:
- The user authentication for a service usage or an asset access is usually executed only at the beginning of the usage/access. This implies that usually, once users have authenticated themselves, the service/asset can be accessed for a specific or unlimited amount of time from their device. As a result, if users leave their device unattended (a common practice), they expose to unauthorized accesses.
- The authentication represents an invasive experience for users, which must actively authenticate themselves, by scanning some object (token, card, etc.), or some characteristic (iris, face, voice, fingerprint, etc.), or by executing specific gestures or specific sequences (signature, password, etc.).
- User security credentials (password, token, or event fingerprint, etc.) are subject to copy or theft by malicious external observers, which can then access the objective service/asset by faking their identity to the one of an authorized user. This is known as spoofing attack.

Some existing authentication have tried to solve or at least minimize the detected authentication problems, without a complete success as they still present major disadvantages, as for example:
- Some services uses password which are valid only for a single use (One-Time Password - OTP), against the standard static authentication paradigm. This allows to overcome standard limitations of the static authentication, such as vulnerability to reply attacks and to brute force discovery. OTPs may be used at each triggering point obtaining a continuous authentication, but each step requires the user intervention to generate a new OTP and insert it to execute the authentication procedure. Moreover the user experience is highly invasive and, furthermore, the token generating the OTPs may include usage fees and/or limitations to the number of usages.
- The "Nymi Always-on-Authentication" includes a bracelet authenticating the user while accessing a service/asset. The bracelet includes biometric reading against a registered biometric profile of the user. The biometric signal is read once when the user wears the bracelet (i.e. it executes a biometric reading only at the time the bracelet is worn) and guarantees authentication until the bracelet is removed by the user. This solution does not allow a continuous update of the user profile (for example, to account for aging); it only guarantees a low degree of security, as the biometric signal is checked only once at the beginning, guaranteeing continuous authentication over a much longer period but without further checks; it only uses the biometric signal as authentication factor so it does not take into account the user status (motion, location), being hence prone to eventual false negatives in the authentication process. Finally, the Nymi's solution uses as biometric signal the electrical heart signal, monitored through an ECG sensor which is expensive and it requires at least two contact points, eventually requiring special movements from the user to be read, resulting in a worst user experience.
- There are other proposed solutions (see patent application documents US2014018870 or CN1540568) which use a biometric signal to profile and identify the users for accessing a service/asset. Biometric can be defined as a set of automated methods that analyze given human traits to identify and authenticate persons. It takes advantage of the fact that there are certain biological or behavioral traits (also called biometric features or characteristics) that are unique and unalterable in each person. Thus, they can be analyzed and measured for creating a biometric profile of the user. These traits are hard to lose, spoof, transfer or forget and are long-lasting. Quite simply, biometric allows identifying an individual not by what the individual possesses or knows, but rather by what the individual is. Biometric features or traits must comply with five basic concepts or foundations, which are universality (each individual must have these biometric traits), uniqueness (different persons must have distinguished biometric traits), permanence (the feature must remain invariant over time), perpetuity (the feature must be permanent over time) and measurability (the feature must allow being quantitatively characterized).

However, these solutions does not take into account the user status (i.e., being prone to eventual higher false negatives), they do not allow to account for the user motion (and location) in the authentication procedure and, depending on the used biometric measuring device and process, the resulting authentication process may result invasive for the user.

Hence, there is a need of a technical solution for authenticating electronic devices users in a highly reliable way which does not present any of the above mentioned problems and that, at the same time, is usable, easy to implement, simple, cheap and which minimum resource and energy consumption.

### SUMMARY

Present invention solves the aforementioned problems by providing a method, device and system for authentication of an user of an electronic device (for example a mobile device or any other electronic device). The user's authentication (for example for a service usage or an asset access) is based on the measurement of a biometric/biotechnological feature of the individual (preferably on the user's pulse signal) and on the detection of the user status.

In other words, in the proposed solution (which will be generally named as PulselD as the pulse is the biometric signal used to identify the user in a preferred embodiment, eventhough other type of biometric signals can be used), a continuous (with opportunistic user's biometric signal measurement) authentication is performed using an user's biometric signal (e.g. the user's pulse signal) as authentication factor with the support of one or more sensors as opportunistic external factors for detecting the user status. In an embodiment, said sensors are motion sensors (as for example, an accelerometer or a gyroscope) and optionally location sensors, allowing to differentiate changes in the system and user motion or location status.

In a preferred embodiment the biometric signal (pulse signal) used for authentication is a Photoplethysmogram (PPG). A photoplethysmogram is an optically obtained plethysmogram, a volumetric measurement of an organ resulting from the variations of the amount of, e.g., blood. A PPG is often obtained by using a pulse oximeter which illuminates the skin through a LED and measures changes in light absorption through a photodiode. A conventional pulse oximeter monitors the perfusion of blood to the dermis and subcutaneous tissue of the skin. Each cardiac cycle, causing a pressure pulse, appears hence as a peak if the oximeter is attached without skin compression. The shape of the PPG waveform differs from subject to subject, and varies with the location and way the pulse oximeter is attached to the user.

According to a first aspect it is provided a method for authentication of an user of a first electronic device (for example for using a certain service or accessing a certain asset or premises), the method comprising the following steps performed by a second electronic device worn by the user (for example a bracelet or any other type of wearable electronic device):
a) receiving from the first electronic device an authentication request message or detecting an authentication triggering event in the second electronic device, and going to step b);
b) determining if the user motion and/or location status has changed (e.g. determining the current motion and/or location status and comparing it with the motion and/or location status of the last time the second electronic device received an authentication request message or an authentication triggering event was detected), and if so (if it has changed) going to step c), otherwise setting the authentication result to the last stored authentication result (stored in the second wireless device, that is, the authentication result is set to the last determined authentication result) and going to step e);
c) measuring an user's biometric signal;
d) determining (and storing) an user's authentication result based at least in the comparison of the measured biometric signal with a biometric signal pattern (e.g. a range of biometric signal values and/or shape) pre-stored for the user for the current user's motion and/or location status (e.g. if the measured signal matches the corresponding pre-stored biometric signal pattern the authentication is positive, otherwise is negative);
e) sending an authentication message including the determined authentication result to the first electronic device.

The biometric signal may be an user's pulse signal, for example a Photoplethysmogram signal obtained using a pulse oximeter.

The user's motion and/or location status mey be for example one of the following: walking, steady or running.

The communications between the first and the second communications devices may be made using Bluetooth technology or any other (usually short-range) wireless technology.

In an embodiment, the authentication of the user is for using a certain service provided by a server and the first electronic device sends the received authentication message to the server providing said certain service or to a firewall which in turn forwards the authentication message to the server. In an embodiment, before step a), an user's authentication for accessing said certain service is performed by the first electronic device using a different authentication factor (that is, an authentication factor different than the biometric signal measured in step c)).

For the determination of a change in the user motion and/or location status may be used information monitored (detected) by one or more motion sensors of the second electronic device and optionally also information monitored by one or more location sensors of the second electronic device (optionally time information may be also used for the determination of an status change).

In an embodiment, for determining the authentication result besides the comparison with the biometric signal pattern, location information provided by one or more location sensors of the second electronic device is also used and, for example, if from said location information it is determined than the user is not inside a pre-established location perimeter (for example a certain perimeter around the first electronic device), the user's authentication result determined in step d) is negative.

The transmission of an authentication request message to the second electronic device may be triggered by certain user interactions (pre-established events) with the first electronic device (e.g. a request of the user to the first electronic device to access a certain service or any click in the service interface).

The authentication triggering event detected by the second electronic device may be one or more of the following: the second electronic device is within a predefined distance range from the first electronic device, a predefined time of the day, a predefined time has lapsed from the last authentication, the user's biometric signal is above a certain predefined threshold, change in the user's motion and/or location status, the second electronic device is being taking off by the user.

If it is the first time the authentication is performed for the user or for any reason there is no last stored authentication result, the last stored authentication result is usually set to a successful authentication result.

Determining whether the user motion and/or location status has changed may comprise:
- (continuously) monitoring user's movements using one or more motion sensors of the second electronic device;
- when receiving the authentication request or detecting the authentication triggering event, determining current user motion and/or location status based at least on the comparison of the monitored user's movements with pre-established motion thresholds;
- comparing the determined current user motion and/or location status with the last stored motion and/or location status.

The first electronic device may be any type of electronic device, for example, a mobile communications device, a computer, a laptop, a tablet, an open/close electronic mechanism (for example of a door)....

According to another aspect it is provided a wearable electronic device for (continuous) authentication of an user of a first electronic device (for example for using a certain service or accessing a certain asset or premises), the wearable electronic device comprising:
- One or more sensors including at least one or more motion sensors (e.g. accelerometer, gyroscope...) and/or one or more location sensors;
- A communications module configured to communicate with the first electronic device;
- A processor configured to:
   - when receiving an authentication request from the first electronic device (for example, when the first electronic device detects certain pre-established service events) through the communications module or detecting an authentication triggering event (using the information provided by one or more sensors, e.g. motion, location or time sensors), determine if the user motion and/or location status has changed since the last time the wearable electronic device received an authentication request or detected an authentication triggering event;
   - if it is determined that the user motion and/or location status has changed, measure an user's biometric signal and determine an user's authentication result based at least in the comparison of the measured biometric signal values with a range of biometric signal values pre-stored for the user for the current user's motion and/or location status and to store the authentication result;
   - if it is determined that the user motion or location status has not changed, set the authentication result to the last stored authentication result;
   - to send a message including the determined authentication result to the first electronic device through the communications module.

According to another aspect it is provided a system for (continuous) authentication of an user of a first electronic device (for example for using a certain service or accessing a certain asset or premises) comprising:
- A first electronic device configured to send an authentication request to a second electronic device worn by the user, every time any of certain pre-established events happen (e.g. on a service provided to the user through the first electronic device);
- The second electronic device comprising:
   - One or more sensors including at least one or more motion sensors and/or location sensors;
   - A communications module configured to communicate with the first electronic device;
   - A processor configured to:
      - receive an authentication request from the first electronic device through the communications module;
      - detect an authentication triggering event using the information provided by one or more sensors;
      - when receiving the authentication request or detecting the authentication triggering event, determining if the user motion or location status has changed since the last time the second electronic device received an authentication request or detected an authentication triggering event;
      - if it is determined that the user motion or location status has changed, to measure an user's biometric signal and to determine an user's authentication result based at least in the comparison of the measured biometric signal values with a range of biometric signal values pre-stored for the user for the current user's motion and/or location status and to store the authentication result;
      - if it is determined that the user motion or location status has not changed, to set the authentication result to the last stored authentication result;
      - to send a message including the determined authentication result to the first electronic device through the communications module.

A last aspect of the invention refers to a computer program product comprising computer program code adapted to perform the method of the invention, when said program code is executed on a computer, a digital signal processor, a field-programmable gate array, an application-specific integrated circuit, a microprocessor, a micro-controller, or any other form of programmable hardware. A non-transitory digital data storage medium is also provided for storing a computer program which comprises instructions causing a computer executing the program to perform the above-described method.

The proposed invention implies many advantages compared to prior art solutions, as for example:
- Continuous authentication. The authentication is performed not only at the beginning of using the service but everytime an authentication triggering event occurs. Moreover, the user is continuously monitored to detect any motion and/or location pattern (user status) change and the biometric signal of the user is read and authenticated (comparing it with the user profile) when the user status change.
- Smooth user experience, transparent to the authentication process.
- Minimization of the biometric measurements thanks to the opportunistic support of the motion (and eventually location and time) sensors.
- Security of the solution: the authentication device cannot be stolen as a change in its status would trigger a new reading of the biometric sensors, not matching now the registered user.
- Possibility to configure the authentication process to work dependently or independently from the location, as a further authentication factor (depending on the presence of location sensors on the bracelet).
- Accounting for the user status, allows to define different ranges for the biometric values associated to the user corresponding to different statuses, allowing to drastically reduce the number of false negatives in the authentication process

### DESCRIPTION OF THE DRAWINGS

To complete the description that is being made and with the object of assisting in a better understanding of the characteristics of the invention, in accordance with a preferred example of practical embodiment thereof, accompanying said description as an integral part thereof, is a set of drawings wherein, by way of illustration and not restrictively, the following has been represented:
Figure 1 shows an overview of the flow diagram of an authentication method according to an embodiment of the invention.
Figure 2 shows an schematic flow chart of an authentication method according to an embodiment of the invention.

### DETAILED DESCRIPTION

The invention describes a method, device and system for, in general terms, authenticating a user of an electronic device (for providing a certain service to the user or accessing a communications network or accessing a certain asset/premises or, even, it can be used as a passcode for a door). Said electronic device can be a mobile communications electronic device (as for example, a mobile telephone, a smartphone) or a computer, a laptop, a tablet... and generally speaking any type of electronic device. For example in the case that the authentication is used to open a door, the electronic device will be the open/close mechanism of the door.

The authentication proposed in the present invention is based on the measurement of an user's biometric signal (the user's pulse in a preferred embodiment) performed by a biometric sensor (for example, a pulse sensor as a pulse oximeter) located in a wearable device (or more specifically, a wearable electronic device) worn by the user. As the biometric signal is the pulse in a preferred embodiment, this authentication solution is generically named as PulselD. In a preferred embodiment, the wearable electronic device may be a bracelet (in other embodiments, it may be an smartwatch, a ring or any other type of wearable electronic device). The user status is taken into account in the authentication; said user status may be determined thanks to sensors (preferably included in the wearable electronic device). In a preferred embodiment said sensors are motion sensors and the user status is a motion user status. In an embodiment, the location of the user is also taken into account; the location status of the user is detected by location sensors, (for example, GPS sensors or any other type as indoor positioning estimated for example by bluetooh sensors) preferably included in the wearable electronic device.

The wearable electronic device is able to communicate with the electronic device requesting authentication (e.g a door access electronic open/close mechanism) by using any known wired or wireless communications technology. In a preferred embodiment, Bluetooth or any other short range wireless technology may be used. In an alternative embodiment, a mobile communications technology such as 2G, 3G, 4G, LTE, 5G... may be used.

In order to perform the authentication, a biometric profile of each user should be created. In a preferred embodiment, the biometric signal (the user's pulse signal) is read by a (smart) bracelet using a pulse detector as for example a pulse oximeter. As stated before, the wearable electronic device (the bracelet) may include also motion sensors (e.g. accelerometer and/or gyroscope) and optionally location sensors and other type of sensors.

The user profiles are created in a learning phase (prior to the authentication operation *per se*)*.* In this learning phase, the required biometric (pulse) patterns (user's pulse profiles) of each of the authorized participants that have to be identified (authenticated) are obtained in this learning phase, and they are stored in a database (an internal database in the bracelet or an external database which the bracelet may access). Besides the pulse, the user biometric profile may be extended with further parameters. The choice is made based on the set of sensors available on the wearable device (bracelet).

In general, a state is associated to the user in a Finite State Machine (FSM) corresponding to the current status on the basis of the combination of the values of the motion (e.g. gyroscope, accelerometer, etc.), and optionally, other sensors (as for example location sensors), and for each defined status, a range of the biometric signals (values and/or shape) is associated to the user which will be the user profile for said status. These statuses may include any kind of statuses and the defined statuses will depend on the type of sensors used to determine the status. For example, if only motion sensors as gyroscope or accelerometer are used, the statuses may be any motion statuses, for instance, walking, running, steady, standing, sit...etc. In an embodiment, said status will be three: still, walking, running. In other embodiments, the still status is divided in "standing" status and "sit down" status. In other embodiments, if location sensors are used, the location is also taken into account for the status determination and the status will depend for example of the proximity of the users to the electronic device (e.g. whether they are in the same room or not) or, generally speaking, the status will also depend on the location of the user.

In another embodiment, time sensors located in the wearable device, can be also used to determine the user status so the time is also taken into account for the user status determination. This way, the status will depend not only on user's motion or location but also on the time of the day or on the time lapsed from the previous status change. Usually, time alone is not used to determine the user's status. That is, the status will not only depend only on time but on time and on the motion and/or location information.

For each user, a profile is created and validated on a trial period (also called learning period or learning phase) to set the proper thresholds for authentications for the biometric signal, corresponding to each defined status. That is, in the learning phase, for each status, a range of values (and/or a range of shapes) of the biometric (pulse) signal is associated to each user (and during the authentication the pulse signal will be measured and compared to this user profile for the current user status, to establish if he/she is success fully authenticated or not). In order to do that, for example, for each authorized user in each status (e.g. walking, running and steady) the pulse is measured to establish the pulse signal (values and shape) for said user in each status (usually this measurement is made several times in order to better adjust the user profile).

Hence, there will be a biometric signal pattern (profile) for a user, for example, while he is running, and a different one while he is standing firm or when he is walking. This way, it will be avoided to not recognize a user while he is running because of the changes the activity is bringing to his biometric values. In other words, taking into account the user status for defining the user profile (defining different ranges for the biometric signal associated to the user corresponding to different statuses) allows to drastically reduce the number of false negatives in the authentication process.

The profile creation may be realized through the use of neural networks to gather time correlations in the read signal and improve the authentication. Once the profile has been created and validated, usually an external server manages the user profiles as an element of the proposed authentication factor for the users. Eventual adjustment of the biometric thresholds (user profile) may be included in the process - on a continuous base - to account for user aging. Alternatively or additionally, the user profile is also stored within the user's bracelet so the bracelet is able to perform the authentication without consulting the server.

The server may be protected by a firewall to ensure the security of the profiles and to limit their access to the strictly needed ones. Furthermore, as it will be explained later, the firewall may start the PulselD authentication process (that may be a two factors authentication because it may use two different types of authentication information to perform the authentication as it will be explained now).

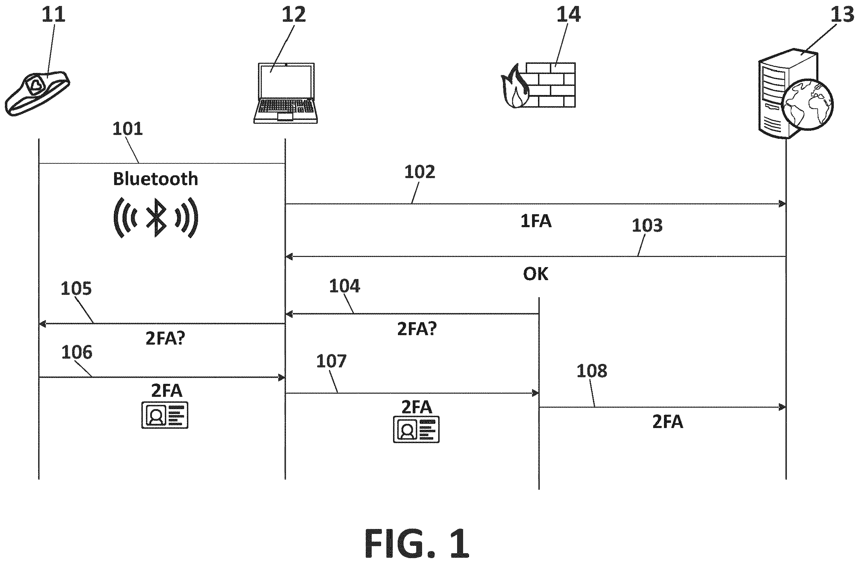

After the learning period (once the user profiles have been created), according to an embodiment of the invention the proposed authentication procedure includes the following steps (this is only a possible embodiment, and not all the cited steps and elements are essential and mandatory in all the embodiments of the present invention). Figure 1 schematically shows the different method steps according to an embodiment of the invention:
- The user wears the wearable electronic device, for example a smart bracelet (11) implementing the proposed authentication (PulseID). The bracelet is connected (101) using any wireless connection (usually through a Bluetooth connection) to the electronic device (12) (also called client device) used by the user to access the service or the asset/premises for which the authentication is requested. This electronic device may be a workstation or generally speaking, it can be any electronic device such as a mobile telephone, a smartphone, or a computer, a laptop, a tablet, an electronic open mechanism, a smart speaker... and any other type of electronic device.
- The bracelet monitors the status of the user (through the motion and eventual location or other sensors).

The user's biometric signal(s) (specifically his/her pulse signal) is not continuously monitored but opportunistically. That is, as it will be explained later, based on different factors (e.g. motion, location or time patterns) it is decided when to read the biometric signal (the pulse), thus saving power and energy consumption in the wearable device (bracelet). In other words, it can be opportunistically decided which are the most suitable moments to read the pulse in advance and provide a reliable measure of the pulse and a prior to need authentication method, thus saving power and energy consumption in the bracelet.
- Optionally, when the user accesses a service though the client device (12) a known standard authentication (usually using an authentication factor different to the pulse signal) is executed. In the embodiment shown in figure 1, the service is provided by a remote server (13), but generally speaking the service will be provided by a service provider which could be any device (including the user's electronic device itself) or any communications network. This standard authentication (using a first authentication factor and that's why it is called in figure 1 1FA) may be performed using any known authentication procedure; for example, a login and password or any user's credential (which will be the first authentication factor) could be asked to the user by an user interface, the user's credential is sent to the service provider (102), the service provider checks that the user's credential is valid and send a successful first authentication (103) to the electronic device. Of course, if this authentication is not successful, the user will not be authorized to access the service.

The communication between the server providing the service and the client device (12) may be made using any communications technology as mobile communications technology (2G, 3G, 4G, LTE...) or any other type of communications technology.

In an embodiment, this first authentication is not performed and the only authentication requested is the authentication disclosed in the present invention (in this case, the authentication will be a one factor authentication). For example, the authentication disclosed in the present invention (and not a standard authentication) is the authentication procedure used to authenticate the user for accessing the service.
- Specific events during the service access may trigger the PulselD authentication. These trigger events may be pre-established events and may be, for instance, a click in a specific part of the service's interface, an interaction with the service after a certain period of time without using the service, an access to a specific sub-service, use of an specific option of the service....

Other possible authentication trigger events may be related with repetitive behavior determined from the motion, location or time patterns detected. For example, if from the user's indoor location, it is detected that the user is close (some radius from) the electronic device (e.g. the computer, the electronic access door...), then the authentication may be triggered. Or for example, if it is detected that authentication is requested usually at the same time of the day or in the same location, the authentication may be triggered at some time in advance said same time of the day or close to said same location automatically. In any of these cases, the reading of pulse and authentication is made several seconds before the user is approaching the electronic device, having the authentication info of the user updated in advance to the claiming of the authentication (e.g. for accesing some asset or premises).

Also, in an embodiment, it can be checked (wit a simple and cheap algorithm) when the user's biometric signal (e.g. pulse) is good enough to make an acceptable authentification (e.g. the pulse signal is strong so the measurement can be appropriately made). If the signal is good enough, an authentication request is triggered and, then, the biometric signal is measured (if the user status has changed). Later, if another authentication request is made and the user status has not changed, the signal is not measured again. This way, it is assured that the biometric signal is measured when the biometric signal is good enough avoiding unsuccessful or wrong measurements, saving cost, time and resources.

This can be mixed with other authentication triggering factors. For example, the authentication can be triggered if the biometric signal is good enough, and the user is close to the electronic device or the time of the day is close to the usual authentication time.

In an embodiment, a change in the user's status (e.g. motion or location status) could trigger itself the Pulse ID authentication.

In an embodiment, based on the motion sensors, it can detected that the wearable electronic device is being taking off. In this case, this will be interpreted as a motion status change and this may trigger an authentication request.

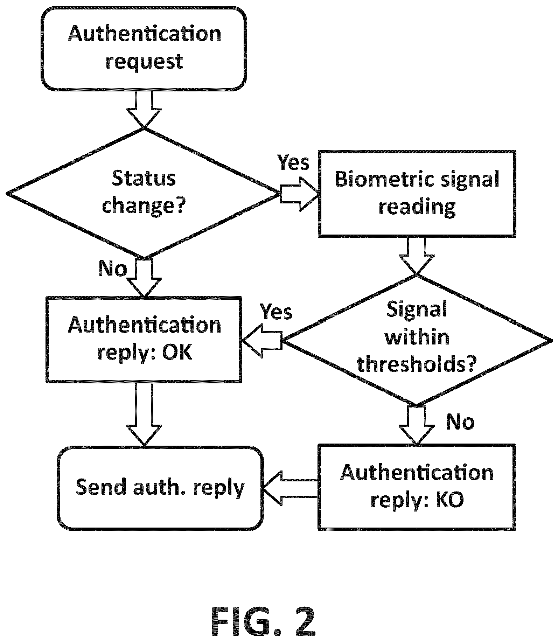

In an embodiment, any interaction of the user with the service (e.g. any click on the service's interface) is an event which triggers the proposed user authentication. In the embodiment shown in figure 1, this authentication procedure is requested (104) by a firewall (14) protecting the remote server (13). In alternative embodiments, the authentication may be requested directly by the remote server (service provider) itself, by the user's electronic device (12) or even by the wearable device itself (11).
- In the case shown in figure 1, when the user's electronic device (12) receives the authentication request (104), a request for authentication (105) is sent to the bracelet (this authentication will be made using a second authentication factor, a biometric signal, and that's why it is called in figure 1 2FA).
- This procedure is shown in figure 2. As stated before, the bracelet monitors the status of the user (through the motion and eventual location sensors) to check if any change occurred to it. The change is the status may be detected for example, determining current user motion and/or location status based at least on the comparison of the information received from the motion /location sensors with pre-established thresholds on the (motion/location) state probabilities. The user status may be managed, for example, using a Finite State Machine (FSM), or an Hidden Markov Model (HMM) so that there are several output probabilities and a different probability on each of them can be used to set the confidence on the motion state. The resulting user motion and/or location status will then be compared with the last stored motion and/or location status.

If, when the bracelet (11) receives an authentication request (105) or the autethentication is triggered by the bracelet itself because of an internal authentication triggering event, any change has occurred to the user status since the last time the authentication was performed (for example, the user was steady and now he starts walking, or he was sit down and now he is standing), the bracelet proceeds to read (measure) the biometric signal to verify the user authentication, by checking if the signal is within the defined thresholds for the user for the new status. This procedure allows the bracelet to predetermine the authentication status for any incoming authentication request in any moment. In case the measured biometric signal is within the defined thresholds for the user and for the new status, the authentication result is positive, otherwise is negative and the user's is not authorized to continue accessing the service. On the other hand, if no status change has been detected since the last authentication, the authentication result is positive.

In an alternative embodiment, the defined thresholds (user profile) are not stored in the bracelet but it another device (for example, the client device or the server), then the bracelet sends the measured biometric signal to the device where the user profile are stored in order to check if said measured biometric signal is between the defined threshold or not. In any case (independently that the user profile is stored in the bracelet or in another device), the thresholds (user profile) depend on the user and said thresholds are trained and obtained independently and specifically for each user.

The authentication result is sent (106) to the client device (12) and, in some embodiments, the client device (12) forwards it to the service provider (e.g. the server 13). If there is a firewall, usually the authentication result is sent to the firewall (107) and from there to the server (108). If the authentication result is negative, the access to the service is not longer allowed for the user.

In an embodiment, if the bracelet includes location sensors, when it receives an authentication request, the bracelet also checks if the location of the user is inside an pre-established perimeter (for example, said perimeter can be several meters around the location of the client device) and, if the location is not inside said perimeter, a negative authentication result is sent to the client device.

For a better understanding, the proposed continuous authentication procedure will be explained for a specific use case. For example, in this use case an user is accessing a service requiring this continuous authentication and the user is seating in front of the client device (12), wearing the bracelet. Usually there is an initial authentication in which it is checked if the initial biometric measurement is between the user's confidence thresholds for the initial user's status; if the initial biometric measurement is between the user's thresholds the initial authentication is positive, otherwise is negative and usually the user's is not authorized to use the service and the authentication procedure ends. After the initial authentication, at every event triggering the proposed continuous authentication, the bracelet (11) receives an authentication request from the client device (12) and the bracelet (11) keeps sending the last stored (positive) authentication to the firewall or server. After a while the user starts walking, bringing around the client device and keeping accessing the service. At the following event triggering continuous authentication, the bracelet determines that the user status has changed and it executes a new reading of the biometric sensor to check if its value is between the thresholds defined for the user in the new status (walking) and, if so, a positive answer is sent for authentication requests until the following status change.

On one side, the designed methodology guarantees the security of the service, as if the user leaves the workstation the continuous authentication will receive a negative answer (if the bracelet includes location sensors, the new location will not match the location allowed for the user) as well as if the bracelet is stolen (the new biometric values will no longer match the profile of the user). On the other side, the designed methodology guarantees a smooth user experience for the user, not needing to actively interact with the authentication process, while still being guaranteed high security authentication. Furthermore, even if the authentication is executed on a continuous base, the number of readings of the biometric values on the bracelet side is minimized to just the status changes, thanks to the system opportunistic support of the motion (and eventual location) sensors.

Besides the use case disclosed above where the user is authenticated for a provided service, e.g. provided by a server (for example, any digital and/or communications service), other possible use cases of the proposed authentication include (but are not limited to):
- House arrest control: PulselD may be used as a control device for house arrest. Current control devices are wearable devices checking their location against the house perimeter, and are hard to remove for the user. On the other hand, current control devices do not authenticate the user. As such, a malicious user can simply remove them and elude their arrest measure by leaving the device in the house perimeter: there is no verification of the user presence, not of their identity authentication. With respect to the current control devices, Pulse ID allows to authenticate the user through their biometric signal, not allowing users to remove the control device. Furthermore, PulselD allows to combine the user location as an authentication factor, allowing to authenticate the user only if located inside the allowed perimeter.
- Weapon usage authorization: Soldiers may be equipped with subcutaneous chips for identification purposes. Subcutaneous chips are passive devices, readable through electromagnetic fields and only from short distances. Weapons may be equipped with security mechanisms requiring user authentication before shooting. PulselD offers a user authentication that can replace the chip, or integrate it. In the latter case, the bracelet may be equipped with a chip reader technology to be used as a mean to authenticate soldiers to use a specific weapon. The user would be identified through the unique code contained in the subcutaneous chip (which could not directly be read by the weapon due to distance constraints), in the case of integrated authentication, adding this factor to the ones offered by the PulselD solution. On the other hand, PulselD alone also offers a biometric based, continuous authentication allowing to authorize the soldier to use the weapon. Further authentication factors may be used, including, for instance, the user location, or user status, as provided by the present solution.

The expressions mobile device or mobile communications device as used herein and throughout this specification, refers to any portable electronic device capable of sending or receiving data using wireless technology, as for example a mobile telephone, a control access door device, an smartphone, a laptop, a PC, a personal Digital Assistant (PDA) a tablet, an i-pad or any other mobile communications device.

Even though in most of the presented embodiments, a bracelet has been mentioned as the wearable electronic device, the solution proposed in the present invention may be applied to any other kind of wearable electronic device as an ankle lace, a necklace, a ring, a collar, an electronic device inserted in clothes worn by the user and generally speaking any type of electronic device.

Even though in most of the presented embodiments, the biometric signal measured from the user is the pulse signal, any other type of biometric signal may be used to the solution proposed in the present invention, as for example voice, iris measurement, face recognition....

The term "comprises" and the derivations thereof (such as "comprising", etc.) must not be understood in an exclusive sense, i.e., these terms must not be interpreted as excluding the possibility that what is described and defined may include additional elements, steps, etc.

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods.

The description and drawings merely illustrate the principles of the invention. Although the present invention has been described with reference to specific embodiments, it should be understood by those skilled in the art that the foregoing and various other changes, omissions and additions in the form and detail thereof may be made therein without departing from the scope of the invention as defined by the following claims. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

## Claims

1. Method for authentication of an user of a first electronic device, the method comprising the following steps performed by a second electronic device worn by the user:
a) receiving from the first electronic device an authentication request or detecting an authentication triggering event in the second electronic device, and going to step b);
b) determining if the user motion and/or location status has changed since the last time the second electronic device received authentication request or detected an authentication triggering event, and if so going to step c), otherwise setting the authentication result to the last stored authentication result and going to step e);
c) measuring an user's biometric signal;
d) determining an user's authentication result based at least in the comparison of the measured biometric signal with a biometric signal pattern pre-stored for the user for the current user's motion and/or location status;
e) sending a message including the determined authentication result to the first electronic device.

2. A method according to claim 1 where the second electronic device is a bracelet.

3. A method according to any of the previous claims where the biometric signal is a user's pulse signal.

4. A method according to claim 3 where the pulse signal is a Photoplethysmogram signal obtained using a pulse oximeter.

5. A method according to any of the previous claims where the user's motion and/or location status is one of the following: walking, steady or running.

6. A method according to any of the previous claims where the communications between the first and the second communications devices are made using Bluetooth technology or any other short-range wireless technology.

7. A method according to any of the previous claims where the authentication of the user is for using a certain service provided by a server and the first electronic device sends the received message including the authentication result to the server providing said certain service or to a firewall which in turn forwards the message to the server.

8. A method according to any of the previous claims where, the authentication of the user is for using a certain service and before step a), an user's authentication for accessing said certain service is performed by the first electronic device using a different authentication factor.

9. A method according to any of the previous claims where, for the determination of a change in the user motion and/or location status is used information monitored by one or more motion sensors of the second electronic device or information monitored by one or more motion sensors and by one or more location sensors of the second electronic device.

10. A method according to any of the previous claims where for determining the authentication result, location information provided by one or more location sensors of the second electronic device is also used and if, from said location information, it is determined than the user is not inside a pre-established location perimeter, the user's authentication result determined in step d) is negative.

11. A method according to any of the previous claims where the transmission of an authentication request to the second electronic device is triggered by certain user interactions with the first electronic device.

12. A method according to any of the previous claims where the authentication triggering event detected by the second electronic device is one or more of the following: the second electronic device is within a predefined distance range from the first electronic device, a predefined time of the day, a predefined time has lapsed from the last authentication, the user's biometric signal is above a certain predefined threshold, change in the user's motion and/or location status, the second electronic device is being taking off by the user.

13. A wearable electronic device for authentication of an user of a first electronic device, the wearable electronic device comprising:
- One or more sensors including at least one or more motion sensors and/or one or more location sensors;
- A communications module configured to communicate with the first electronic device;
- A processor configured to:
- when detecting an authentication triggering event or receiving an authentication request from the first electronic device through the communications module, determine if the user motion and/or location status has changed since the last time the wearable electronic device received an authentication request or detected an authentication triggering event;
- if it is determined that the user motion and/or location status has changed, measure an user's biometric signal and determine an user's authentication result based at least in the comparison of the measured biometric signal values with a range of biometric signal values pre-stored for the user for the current user's motion and/or location status and to store the authentication result;
- if it is determined that the user motion or location status has not changed, set the authentication result to the last stored authentication result;
- to send a message including the determined authentication result to the first electronic device through the communications module.

14. A system for authentication of an user of a first electronic device comprising:
- A first electronic device configured to send an authentication request to a second electronic device worn by the user, every time any of certain pre-established events happen;
- The second electronic device comprising:
- One or more sensors including at least one or more motion sensors and/or location sensors;
- A communications module configured to communicate with the first electronic device;
- A processor configured to:
- receive an authentication request from the first electronic device through the communications module;
- detect an authentication triggering event using the information provided by one or more sensors;
- when receiving the authentication request or detecting the authentication triggering event, determining if the user motion or location status has changed since the last time the second electronic device received an authentication request or detected an authentication triggering event;
- if it is determined that the user motion or location status has changed, to measure an user's biometric signal and to determine an user's authentication result based at least in the comparison of the measured biometric signal values with a range of biometric signal values pre-stored for the user for the current user's motion and/or location status and to store the authentication result;
- if it is determined that the user motion or location status has not changed, to set the authentication result to the last stored authentication result;
- to send a message including the determined authentication result to the first electronic device through the communications module.

15. A non-transitory digital data storage medium for storing a computer program which comprises instructions causing a computer executing the program to perform the method according to any of the claims 1-12.
